# FASCICULE DE BREVET EUROPEEN

(11) **EP 2 726 486 B1**
(45) Date de publication et mention de la délivrance du brevet: **22.04.2015**
(21) Numéro de dépôt: 12731055.5
(22) Date de dépôt: 24.05.2012
(51) Int. Cl.: C07D 493/04, A61K 31/34, A61K 31/4025, A61Q 19/00

(54) **NOUVEAUX ESTERS DE DÉRIVÉS N-ACYLÉS D'ACIDES AMINÉS ET D'ISOSORBIDE, PROCÉDÉ POUR LEUR PRÉPARATION, ET UTILISATION EN COSMÉTIQUE ET COMME MÉDICAMENT**
NEUE ESTER VON N-ACYLIERTEN AMINOSÄURE- UND ISOSORBIDDERIVATEN, VERFAHREN ZU IHRER HERSTELLUNG SOWIE IHRE VERWENDUNG IN DER KOSMETIK UND ALS ARZNEIMITTEL
NOVEL ESTERS OF N-ACYLATED DERIVATIVES OF AMINO ACIDS AND ISOSORBIDE, METHOD FOR PREPARING SAME, AND USE THEREOF IN COSMETICS AND AS DRUG

(30) Priorité: 30.06.2011 FR 1155900
(43) Date de publication de la demande: 07.05.2014
(73) Titulaire: Societe d'Exploitation de Produits pour Les Industries Chimiques Seppic, 75007 Paris (FR)
(72) Inventeur: BENATTAR, André, F-81100 Castres (FR); CATTUZZATO, Laetitia, F-81100 Castres (FR); DUMONT, Sandy, F-81200 Caucalieres (FR); GARCEL, Stéphanie, F-81100 Castres (FR); GUILBOT, Jerôme, F-81100 Castres (FR); KERVERDO, Sébastien, F-94300 Vincennes (FR); ROLLAND, Hervé, F-81100 Castres (FR)
(74) Mandataire: Conan, Philippe Claude
(86) Numéro de dépôt international: PCT/FR2012/051167
(87) Numéro de publication internationale: WO 2013/001192

(56) Documents cités:
- WO-A2-2011/059866
- FR-A1- 2 883 171
- MASAHIKO OKADA ET AL: "Biodegradable polymers based on renewable resources. V. Synthesis and biodegradation behavior of poly(ester amide)s composed of 1,4:3,6-dianhydro-D-glucitol, ?-amino acid, and aliphatic dicarboxylic acid units", JOURNAL OF APPLIED POLYMER SCIENCE, vol. 81, no. 11, 12 septembre 2001 (2001-09-12), pages 2721-2734, XP055014068, ISSN: 0021-8995, DOI: 10.1002/app.1718
- Z. GOMURASHVILI, H. R. KRICHELDORF & R. KATSARAVA: JOURNAL OF MACROMOLECULAR SCIENCE, PURE AND APPLIED CHEMISTRY, vol. A37, no. 3, 2000, pages 215-227, XP009154634, DOI: 10.1081/MA-100101089

## Description

La présente invention concerne de nouveaux produits chimiques, et de nouvelles compositions chimiques, destinés à la prévention et/ou au traitement des signes visibles du dysfonctionnement du système veineux et/ou de l'altération de la perméabilité vasculaire de la peau humaine.

La peau humaine constitue la première image offerte au regard d'autrui, et par conséquent, l'amélioration de son aspect est un sujet de préoccupation constant pour les êtres humains. La peau est le reflet d'un état de bien-être, souvent associé à la jeunesse, et *a contrario* d'un état de fatigue et/ou de vieillissement.

Un bon fonctionnement de la microcirculation sanguine constitue un des facteurs essentiels qui régissent le bon état esthétique cutané. Le phénomène de vieillissement se traduit notamment par une diminution du nombre, de la taille et de la fonctionnalité des vaisseaux dermiques qui assurent le fonctionnement de la microcirculation sanguine, ont tendance à devenir moins nombreux et plus fragiles.

Cette raréfaction des vaisseaux capillaires sous-cutanés entraine une moindre oxygénation de la peau et une diminution de l'apport en nutriments (oligo-éléments et vitamines), ce qui se traduit chez l'être humain âgé par une pâleur faciale chronique (références bibliographiques (1), (2) et (3). De même, tout dysfonctionnement du système veineux, qui se caractérise par un ralentissement de la circulation sanguine, produit le même phénomène d'oxygénation insuffisante du tissu connu sous le nom d'hypoxie.

La notion de perméabilité vasculaire décrit le rôle joué par de petits vaisseaux sanguins (artérioles, veinules ou micro-vaisseaux) pour apporter une fonction de barrière entre le sang circulant dans lesdits petits vaisseaux sanguins et les tissus, plus particulièrement les tissus cutanés. Lorsque la perméabilité vasculaire est altérée sous l'effet du vieillissement et/ou de phénomènes inflammatoires et/ou de stress d'origine extérieure, les tissus alors en situation d'hypoxie sont enflammés, ce qui se manifeste par l'apparition de phénomènes de rougeurs exacerbés, pouvant aller jusqu'à la formation d'oedèmes sur la zone cutanée concernée. Les vaisseaux peuvent par ailleurs se dilater, voire se rompre, entraînant la formation de télangiectasies.

L'endothélium est un tissu dont la fonction première est de contenir le sang à l'intérieur des vaisseaux sanguins, en permettant l'échange de substances nutritives avec le milieu intérieur. Il est constitué par les cellules endothéliales et par les cellules musculaires, qui agissent comme des « filtres » moléculaires pour permettre cet échange de substances nutritives, dont la fonction est de contrôler la coagulation sanguine et la vasomotricité de l'individu. Les médiateurs humoraux, les hormones, les cytokines ou les facteurs de croissance, constituent des contraintes biochimiques qui agissent sur l'activation des cellules endothéliales. Les cellules endothéliales sont sensibles au stress oxydatif, provoqué par une présence accrue de dérivés oxygénés, comme par exemple des ions superoxydes, des péroxydes d'hydrogène, des radicaux hydroxyles, qui dépassent les capacités régulatrices du système anti-oxydant naturel (superoxydismutase, catalase...), ce qui se traduit notamment par une diminution d'oxygène disponible dans les cellules endothéliales à savoir d'un phénomène d'hypoxie desdites cellules endothéliales, et par conséquent par une baisse de la production d'Adénine TriPhosphate (ATP) dans lesdites cellules endothéliales.

Selon la littérature (référence bibliographique (4) Janssens, « effect of venotropic drugs on the respiratory activity of isolated mitochondria and in endothelial cells », in British Journal of Pharmacology (2000) 130, 1513-1524*),* les insuffisances veineuses, résultant de dysfonctionnement du système veineux et/ou de l'altération de la perméabilité vasculaire, qui se traduisent par la diminution de l'apport sanguin artériel à un organe (ou ischémie), laquelle entraine essentiellement une baisse de l'oxygénation des tissus de l'organe en dessous de ses besoins pour le placer en situation d'hypoxie et par conséquent une diminution de la production d'ATP par les cellules endothéliales.

Selon l'organisation des cellules endothéliales dans les organes, l'endothélium remplit une fonction spécifique dudit organe. De ce fait, lorsqu'elles sont soumises à une contrainte mécanique ou biochimique, les cellules endothéliales génèrent des réponses aux stimuli de natures différentes (exposition aux rayonnements UltraViolets, variation importante de température et/ou d'hygrométrie, pollution, ...), qui ont des conséquences macroscopiques différentes. Ainsi, un dysfonctionnement du système veineux et/ou de l'altération de la perméabilité vasculaire, généré et/ou exacerbé par des contraintes mécaniques et/ou biochimiques, peut se traduire par une situation d'hypoxie dans la région du contour de l'oeil se manifestant par l'apparition d'oedème de caractère non inflammatoire, et notamment par l'apparition de cernes et/ou de poches sous les yeux, ou au niveau des membres inférieurs, par l'apparition de sensations de lourdeur des jambes se traduisant notamment par un gonflement du mollet et/ou des pieds et/ou des chevilles.

La région du contour de l'oeil se caractérise par une innervation dense et par une peau fine, peu riche en lipides cutanés, se montrant alors très sensible aux stress extérieurs (état de fatigue, manque de sommeil, exposition aux UV, tabac, alcool,...) et aux différentes contraintes mécaniques et biochimiques. Les dysfonctionnements du système veineux et/ou l'altération de la perméabilité vasculaire qui se traduisent par une vasodilatation ou une congestion des capillaires sanguins dans cette zone particulière du contour de l'oeil sont également plus visible du fait de la minceur de la peau. Lorsque la vasodilatation ou la congestion des capillaires sanguins présents sous les yeux perdurent, ces phénomènes engendrent des sensations d'inconfort persistantes et provoquent l'apparition de cernes et/ou de poches sous les yeux qui revêtent alors un caractère inesthétique. Le vieillissement cutané se traduit également par une diminution du nombre, de la taille et de la fonctionnalité des vaisseaux dermiques, ce qui entraîne une diminution de l'apport nutritionnel et de l'éclat du teint. Ces phénomènes sont également confortés par une circulation lymphatique ralentie dans cette zone du contour de l'oeil.

En ce qui concerne le phénomène ou la sensation de lourdeur au niveau des membres inférieurs, et notamment le phénomène dit de « jambes lourdes », il est ressenti par les sujets qui présentent des dysfonctionnements du système veineux et/ou une altération de la perméabilité vasculaire, déclenchés ou aggravés par des facteurs liés à l'hérédité, à la sédentarité, à une station debout prolongée, à l'exposition à la chaleur, à l'abus de tabac ou d'alcool. Ce phénomène se caractérise par une dilatation des veines, et se manifeste par l'apparition de douleurs, de fourmillements et de gonflement du mollet, des pieds et des chevilles.

Il existe donc un besoin de disposer de solutions satisfaisantes pour prévenir et/ou traiter les diminutions de productions de l'ATP par les cellules endothéliales sous l'effet de stress oxydants, de façon à prévenir et/ou traiter les dysfonctionnements du système veineux et/ou l'altération de la perméabilité vasculaire qui se traduisent par l'hypoxie des cellules endothéliales du corps humain et par des effets inesthétiques, comme par exemple les cernes et/ou les poches péri-oculaires et le phénomène de « jambes lourdes ».

Les produits de maquillage constituent une solution qui permet de masquer, d'atténuer les défauts apparents de la peau, et peuvent présenter une solution à la présence de cernes et de poches dans la zone péri-oculaire. Les fonds de teint procurent un aspect mat à la peau et permettent d'unifier son teint. Mais ces solutions cosmétiques ne permettent de traiter que les conséquences apparentes des dysfonctionnements du système veineux ou de l'altération de la perméabilité vasculaire sur la seule zone péri-oculaire sans traiter leurs causes. De plus, l'utilisation de ces compositions de maquillage présentent l'inconvénient de conférer à la peau un aspect non naturel et certaines d'entre elles sont difficiles à étaler et peuvent provoquer un assèchement de la peau sur le long terme.

Une autre solution consiste à favoriser la production de monoxyde d'azote par les mitochondries des cellules sujettes au phénomène d'hypoxie. Le monoxyde d'azote est une molécule connue qui est libérée notamment par les cellules endothéliales ce qui permet de provoquer le phénomène de vasodilatation et par conséquent un accroissement du débit sanguin. La publication internationale WO2008/141296A1 décrit un procédé de traitement de l'hypoxie de tissus de mammifères en exposant lesdits tissus à des rayonnements électromagnétiques dans la partie visible du spectre de la lumière, de façon à favoriser la production de monoxyde d'azote par les mitochondries des tissus exposés à ces rayonnements. La publication FR 2 883 171 A1 décrit l'utilisation d'agents favorisant la production de monoxyde d'azote dans et/ou sur la peau, choisis parmi des donneurs ou des précurseurs de monoxyde d'azote (comme par exemple les composés comprenant des substituants nitro ou nitroso, des oximes, l'hydroxylamine, la N-hydroxy guanidine et ses sels, des métaux de transition nitrosylés, ...), des agents permettant la libération non polymérique de monoxyde d'azote dans l'organisme (comme par exemple des acides aminés, des peptides), des agents permettant la stimulation de la synthèse et/ou de l'activité monoxyde d'azote synthase (NOS) comme par exemple des interleukines, des lipopolysaccharides, l'acide L-glutamique, l'acide arachidonique. Cette approche par la mise en oeuvre de solutions visant à générer une augmentation de la production de monoxyde d'azote dans l'organisme présente cependant comme inconvénients de ne concerner que la stimulation du phénomène de vasodilatation et d'induire des risques de dérégulation de la balance vasodilatation / vasoconstriction ; l'alternance et l'équilibre des deux phénomènes étant à respecter pour conserver et/ou retrouver un fonctionnement du système veineux et/ou une perméabilité vasculaire équilibrés.

Une autre solution consiste à favoriser la chélation des ions Fe³⁺, présents dans l'hémosidérine qui est un pigment résultant de la dégradation de l'hémoglobine accumulée dans les vaisseaux capillaires du fait d'un ralentissement de la microcirculation sanguine dans la région péri-oculaire. La publication internationale WO2008035152 A1 décrit plusieurs agents chélateurs des ions ferriques efficaces et ne présentant pas de problèmes d'irritation oculaire lors de l'application d'une formulation les contenant sur la zone à préserver ou à traiter.

Parmi ces agents, on peut citer : la 3-hydroxy-2-methyl-4-pyrone (ou maltol), l'éthyl maltol, l'octopirox, le ciclopirox, le rilopirox, l'acide gallique, les esters de l'acide gallique, l'acide kojique, les dérivés de l'acide kojique. Cette solution, outre qu'elle met en oeuvre des composés soit en mélange dans des extraits végétaux soit obtenus à l'issue de procédés multi-étapes non adaptés à l'industrie cosmétique, ne permet de traiter que les conséquences apparentes des dysfonctionnements du système veineux ou de l'altération de la perméabilité vasculaire sur la seule zone péri-oculaire sans traiter leurs causes.

La publication internationale WO2011/059866 décrit des dérivés monoalkanoyl et dialkanoyl de l'isosorbide ainsi que l'utilisation de compositions cosmétique comprenant de tels composés pour réduire les manifestations du vieillissement de la peau des mammifères, comme par exemple l'atrophie de la peau desdits mammifères, et pour prévenir ou retarder l'apparition de cernes sous les yeux.

La demande de brevet japonais No 2000-229921 décrit l'utilisation de polyols esters de N-acylamino acides comme agent tensioactifs performants (paragraphe [0003] de ladite demande). La publication internationale WO2010034917 décrit les mono-esters et les di-esters de polyols du N-(ω-undecylenoyl) phenylalanine et leurs utilisations comme agents éclaircissants de la peau humaine.

La littérature (référence bibliographique (5) M. Okada et al., J.Appl.Polym.Sci., 2001, 81(11), pages 2721-2734, et référence bibliographique (6) Z. Gomurashvili et al., J.Macromol.Sci., Pure Appi.Chem., 2000, A37(3), 215-227) divulgue la synthèse de polymères obtenus par polycondensations de sels de l'acide p-toluène de dérivés d'isohexide avec des esters d'acides dicarboxyliques.

A notre connaissance, aucun ester de dérivés N-acyl aminoacides n'a été décrit comme capable de prévenir et/ou de traiter les diminutions de productions de l'ATP par les cellules endothéliales soumises à des stress oxydants. Par conséquent, à notre connaissance, aucun ester de dérivés N-acyl aminoacides n'a été décrit comme capable de prévenir et/ou de traiter les dysfonctionnements du système veineux et/ou de l'altération de la perméabilité vasculaire. De même, à notre connaissance, aucun ester de dérivés N-acyl aminoacides n'a été décrit comme capable de prévenir l'apparition et/ou de diminuer les effets inesthétiques générés par l'hypoxie des cellules endothéliales du corps humain, comme par exemple les cernes et/ou les poches péri-oculaires et le phénomène de « jambes lourdes ».

La demanderesse s'est donc attachée à développer une solution technique nouvelle, consistant en de nouveaux esters de dérivés N-acyl aminoacides, qui permettent de prévenir et/ou de ralentir la baisse de la production de l'ATP par les cellules endothéliales soumises à des stress oxydants, de façon à prévenir et/ou à traiter les dysfonctionnements du système veineux et/ou de l'altération de la perméabilité vasculaire, et par conséquent de prévenir l'apparition et/ou de diminuer les effets inesthétiques générés par l'hypoxie des cellules endothéliales du corps humain, comme par exemple les cernes et/ou les poches péri-oculaires et le phénomène de « jambes lourdes ».

C'est pourquoi, selon un premier aspect, l'invention a pour objet un composé de formule (I) : 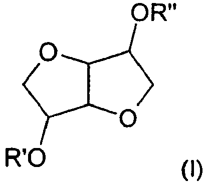 dans laquelle R' et R", identiques ou différents, représentent :
- Soit un atome d'hydrogène ;
- Soit un radical monovalent de formule (IIa) : 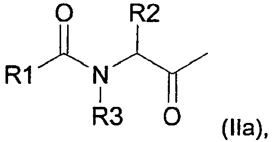
dans laquelle :
- R1 représente un radical aliphatique, saturé ou insaturé, linéaire ou ramifié, comportant de 7 à 30 atomes de carbone,
- R2 représente un atome d'hydrogène ou un radical choisi parmi les radicaux méthyle, isopropyle, isobutyle, 1-méthyl propyle, hydroxyméthyle, 1-hydroxy éthyle, thiométhyle, 2-méthylthio éthyle, 4-aminobutyle, 3-guanidino propyle, 3-uréido propyle, (1-amino carbonyl) méthyle, carboxy méthyle, 2-carboxy éthyle, 2-(amino carbonyl) éthyle, benzyle, 4-hydroxy benzyle, 3,4-dihydroxy benzyle, [1H-indol-3-yl] méthyle, (1H-imidazol-4-yl) méthyle, 3-amino propyle, et
- R3 représente un atome d'hydrogène ou un radical méthyle ;
- Soit un radical monovalent de formule (IIb) : 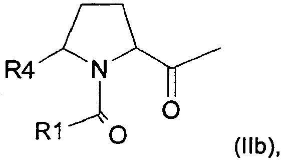
dans laquelle :
- R1 est tel que défini dans la formule (IIa) et
- R4 représente un atome d'hydrogène ou un radical hydroxy,
étant entendu qu'au moins un des radicaux R' ou R" ne représente pas un atome d'hydrogène et que lorsqu'aucun des radicaux R' et R" ne représente un atome d'hydrogène, R' et R" sont identiques.

Selon un premier aspect particulier de la présente invention, le composé de formule (I) telle que définie ci-dessus est plus particulièrement choisi parmi les esters d'isosorbide dérivés des acides aminés suivants : la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la glutamine, la cystéine, la méthionine, l'hydroxyproline, l'hydroxylysine, la sarcosine ou l'ornithine.

Selon un aspect particulier de la présente invention, celle-ci a pour objet un composé de formule (I) telle que définie précédemment, dans laquelle le radical R₁ comporte de 7 à 22 atomes de carbone.

Selon cet aspect particulier, dans la définition du radical de formule (IIa) ou du radical de formule (IIb), le radical R₁-C(=O)- représente principalement un radical choisi parmi les radicaux octanoyle, décanoyle, ω-undécylènoyle, dodécanoyle, tétradécanoyle, hexadécanoyle, octadécanoyle, eicosanoyle, docosanoyle, 9-octadécènoyle, éicosènoyle, 13-docosènoyle, 9,12-octadécadiènoyle ou 9,12,15-octadécatriénoyle,

Selon un autre aspect particulier de la présente invention, celle-ci a pour objet un composé de formule (I) telle que définie précédemment, dans laquelle les radicaux R' et R", identiques ou différents, représentent :
- Soit un atome d'hydrogène ;
- Soit un radical monovalent de formule (IIa), dans laquelle R1 et R3 sont tels que définis précédemment et R2 représente un radical choisi parmi les radicaux méthyle, isopropyle, isobutyle, 1-méthyl propyle ou benzyle,
- Soit un radical monovalent de formule (IIb), dans laquelle R1 est tel que défini précédemment et R4 représente un atome d'hydrogène.

Selon cet aspect particulier de la présente invention, le composé de formule (I) telle que définie ci-dessus est plus particulièrement choisi parmi les esters d'isosorbide dérivés des acides aminés suivants : l'alanine, la valine, la proline, la leucine, la phénylalanine, l'isoleucine.

Selon un autre aspect particulier, l'invention a pour objet un composé de formule (la) : 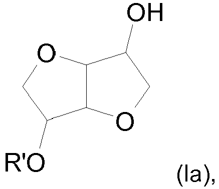 correspondant à la formule (I) telle que définie précédemment, dans laquelle R" représente un atome d'hydrogène, et tout particulièrement :
- Le composé de formule (Ia1) : 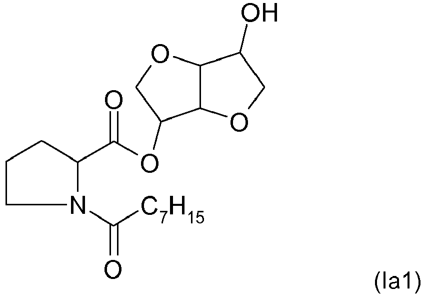
- Le composé de formule (Ia2) : 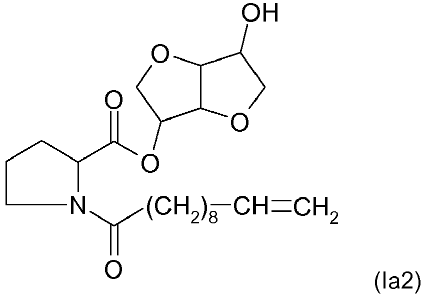
- Le composé de formule (Ia3) : 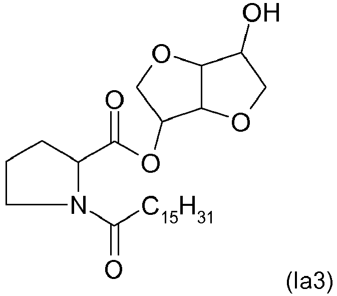
- Le composé de formule (Ia4) : 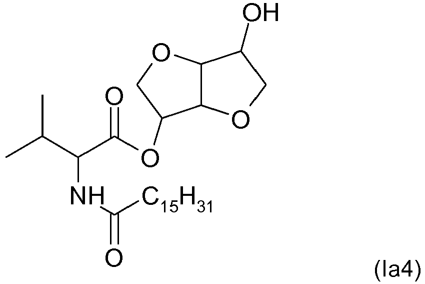
- Ou le composé de formule (Ia5) :

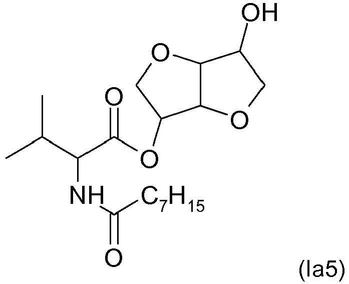

Selon cet aspect particulier de la présente invention, les composés de formules (Ia1), (Ia2), (Ia3), (Ia4), et (Ia5), sont plus particulièrement respectivement choisis parmi:
Le N-octanoyl prolinate de monoisosorbide,
Le N-(ω-undecylènoyl) prolinate de monoisosorbide,
Le N-hexadécanoyl prolinate de monoisosorbide,
Le N-hexadécanoyl valinate de monoisosorbide, ou
Le N-octanoyl valinate de monoisosorbide,

Selon un autre aspect particulier, l'invention a pour objet un composé de formule (Ib) : 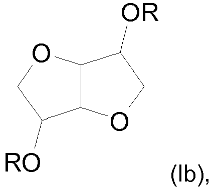 correspondant à la formule (I) telle que définie précédemment, dans laquelle les radicaux R' et R" sont identiques et sont représentés par le radical R, et tout particulièrement :
- Le composé de formule (Ib1) : 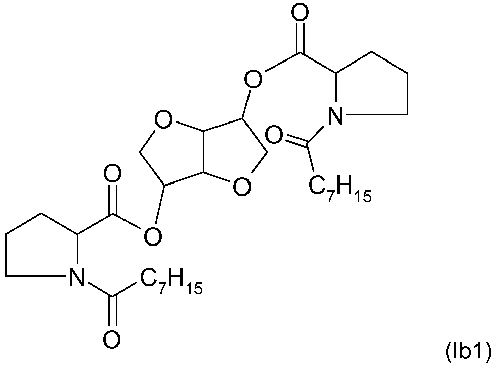
- Le composé de formule (Ib2) : 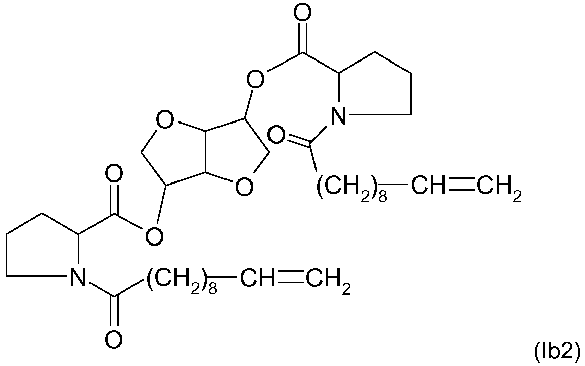
- Le composé de formule (Ib3) : 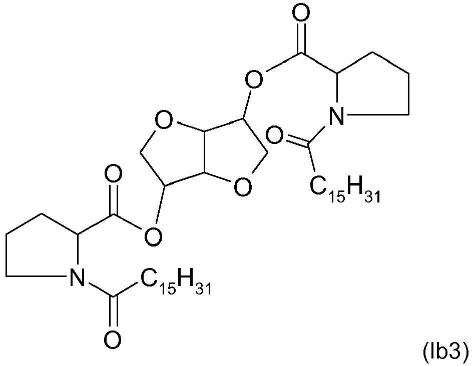
- Le composé de formule (Ib4) : 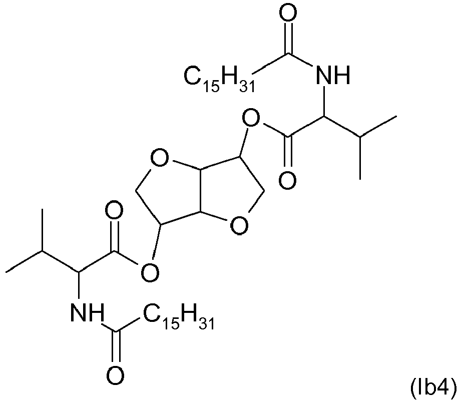
- Ou le composé de formule (Ib5) :

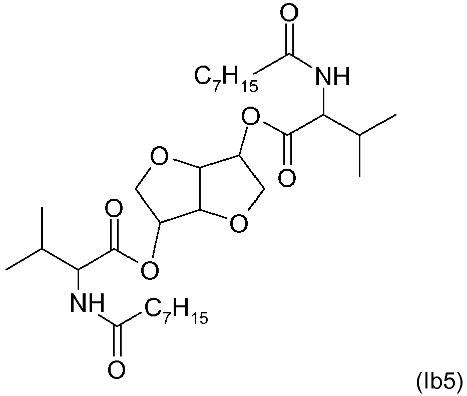

Selon cet aspect particulier de la présente invention, les composés de formules (Ib1), (Ib2), (Ib3), (Ib4), et (Ib5), sont plus particulièrement respectivement choisis parmi :
Le N-octanoyl prolinate de diisosorbide,
Le N-(ω-undecylènoyl) prolinate de diisosorbide,
Le N-hexadécanoyl prolinate de diisosorbide,
Le N-hexadécanoyl valinate de diisosorbide, ou
Le N-octanoyl valinate de diisosorbide,

L'invention a aussi pour objet un procédé de préparation d'un composé de formule (I) telle que définie précédemment, comprenant :
- Une étape a) d'estérification :
   - Soit d'un composé de formule (IIIa) : 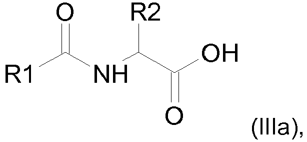 dans laquelle R1 et R2 sont tels que définis dans la formule (IIa),
   - Soit d'un composé de formule (IIIb) : 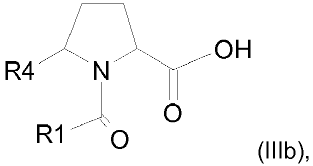
   dans laquelle R₁ et R4 sont tels que définis pour la formule (IIb), avec l'isosorbide de formule (IV) : 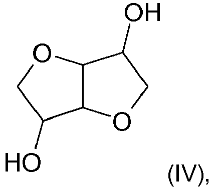 pour obtenir, soit le composé de formule (la), soit le composé de formule (Ib), soit un mélange (M) du composé de formule (la) et du composé de formule (Ib) ; et si nécessaire ou si désiré,
- Une étape b) de séparation des composés de formule (la) et de formule (Ib), à partir du dudit mélange (M) obtenu à l'étape (a).

Les composés de formules (IIIa) et (IIIb) sont connus ou sont synthétisables par N-acylation des α-amino acides correspondant selon des méthodes connues de l'homme du métier.

Dans le procédé tel que défini ci-dessus, le rapport molaire composé de formule (IIIa) ou de formule (IIIb) sur isosorbide de formule (IV) est généralement compris entre 3/1 et 1/5, plus particulièrement entre 1/1 et 1/5, et encore plus particulièrement entre 1/1 et 1/3.

Dans le procédé tel que défini ci-dessus, l'étape b) de séparation des composés de formule (la) et de formule (Ib) est mise en oeuvre par les méthodes classiques de séparation connues de l'homme du métier.

L'invention a aussi pour objet un procédé de préparation d'un composé de formule (I) telle que définie précédemment comprenant :
- Une étape a1) d'estérification, soit d'un composé de formule (IIIa) 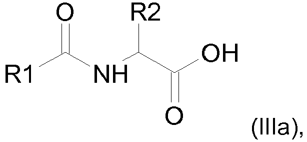 dans laquelle R1 et R2 sont tels que définis dans la formule (IIa), soit d'un composé de formule (IIIb) 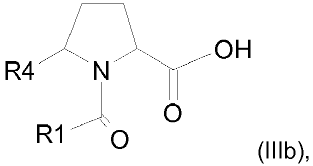 dans laquelle R₁ et R4 sont tels que définis pour la formule (IIb), avec un alcool de formule (V) :

   R5-OH (V),

   dans laquelle R5 représente un radical aliphatique linéaire comportant de 1 à 4 atomes de carbone, pour former :
- Soit un composé de formule (VIa) : 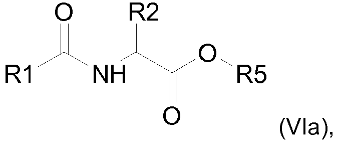 dans laquelle R1, R2 et R5 sont tels que définis précédemment,
- Soit un composé de formule (Vlb) : 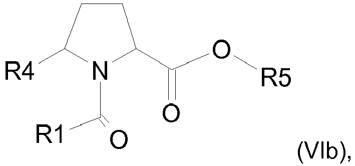 dans laquelle R1, R4 et R5 sont tels que définis précédemment ;
- Une étape a2) de trans-estérification du composé de formule (VIa) ou du composé de formule (Vlb) obtenu à l'étape a1), par réaction avec l'isosorbide de formule (IV), pour obtenir, soit le composé de formule (la), soit le composé de formule (Ib), soit un mélange (M) du composé de formule (la) et du composé de formule (Ib) ; et si nécessaire ou si désiré,
- La mise en oeuvre de l'étape b).

Dans le procédé objet de la présente invention tel que défini ci-dessus, l'étape a1) est généralement réalisée à une température d'environ comprise entre 60°C et 120°C, sous gaz inerte, et en présence d'un système catalytique acide. Par système catalytique acide on désigne les acides forts comme l'acide sulfurique, l'acide chlorhydrique, l'acide phosphorique, l'acide nitrique, l'acide hypophosphoreux, l'acide méthanesulfonique, l'acide para-toluène sulfonique, l'acide trifluorométhane sulfonique, ou les résines échangeuses d'ions acides.

Dans l'étape a1) du procédé objet de la présente invention tel que défini ci-dessus, le rapport molaire composé de formule (IIIa) ou de formule (IIIb) sur alcool de formule (V) est généralement compris entre 1/1 et 1/10, plus particulièrement entre 1/1 et 1/8, et encore plus particulièrement entre 1/2 et 1/8.

Dans le procédé objet de la présente invention tel que défini ci-dessus, l'étape a2) de trans-estérification de l'ester de formule (VIa) et/ou de formule (Vlb) obtenu à l'étape a1) est généralement réalisée à une température d'environ comprise entre 80°C et 180°C, plus particulièrement comprise entre 100°C et 150°C, encore plus particulièrement entre 120°C et 150°C, sous gaz inerte, et en présence d'un système catalytique acide tel que précédemment décrit, et avec distillation sous vide de l'alcool de formule (VI) formé in-situ.

Dans l'étape a2) de procédé, le rapport molaire composé de formule (VIIa) et/ou de formule (Vllb) sur isosorbide de formule (V) est compris entre 3/1 et 1/5, plus particulièrement entre 1/1 et 1/5, et encore plus particulièrement entre 1/1 et 1/3.

L'invention a aussi pour objet une composition (C1) comprenant pour 100% de sa masse :
- De 99% massique à 20% massique, plus particulièrement de 99% massique à 50 % massique, et encore plus particulièrement de 95% massique à 75% d'au moins un composé de formule (la) telle que définie précédemment, et
- De 1% massique à 80% massique, plus particulièrement de 1% massique à 50% massique, et encore plus particulièrement de 5% massique à 25% massique d'au moins un composé de formule (Ib) telle que définie précédemment.

Selon un aspect particulier, dans la composition (C1) objet de la présente invention, le composé (la) est sélectionné parmi les composés de formule (Ia1), (Ia2), (Ia3), (Ia4), ou (Ia5) telles que définies précédemment et le composé (Ib) est sélectionné parmi les composés de formule (Ib1), (Ib2), (Ib3), (Ib4) ou (Ib5) telles que définies précédemment.

La composition (C₁) objet de l'invention peut être préparée par diverses voies.

Une première voie de préparation de la composition (C₁) objet de l'invention consiste à mélanger dans les proportions massiques souhaitées, le composé de formule (la) tel que définie ci-dessus ou le mélange de composés de formule (la), avec le composé de formule (Ib) telle que définie ci-dessus, ou le mélange de composés de formule (Ib).

Une seconde voie de préparation de la composition (C₁) objet de l'invention consiste à mettre en oeuvre le procédé de préparation du composé de formule (I) tel que décrit précédemment, en faisant réagir dans les proportions souhaitées, l'isosorbide de formule (IV) avec le composé de formule (IIIa) ou de formule (IIIb) ou un mélange de composés de formule (IIIa) et de formule (IIIb).

Une troisième voie de préparation de la composition (C1) objet de l'invention consiste à mettre en oeuvre la variante du procédé de préparation du composé de formule (I) telle que décrite précédemment, en faisant réagir dans les proportions souhaitées, l'isosorbide de formule (IV) avec le composé de formule (VIa) ou de formule (Vlb) ou un mélange de composés de formule (VIa) et de formule (Vlb).

L'invention a aussi pour objet l'utilisation du composé de formule (I) ou de la composition (C1) telles que définies précédemment, comme agent actif cosmétique, pour prévenir et/ou limiter les effets inesthétiques générés par l'hypoxie des cellules endothéliales du corps humain et plus particulièrement ceux générés par les cernes, les poches péri-oculaires et/ou les jambes lourdes.

Le composé de formule (I) et la composition (C1) objets de la présente invention peuvent être administrés par voie orale, topique ou parentérale.

L'invention a aussi pour objet une formulation cosmétique à usage topique caractérisée en ce qu'elle comprend au moins un excipient cosmétiquement acceptable et une quantité efficace du composé de formule (I) ou de la composition (C1) telle que définie précédemment.

L'expression "à usage topique" utilisée dans la définition de la formulation cosmétique telle que décrite ci-dessus, signifie que ladite formulation est mise en oeuvre par application sur la peau, qu'il s'agisse d'une application directe dans le cas d'une formulation cosmétique ou d'une application indirecte par exemple dans le cas d'un produit de soin corporel sous forme de lingette en textile ou en papier ou de produits sanitaires destinés à être en contact avec la peau.

L'expression « cosmétiquement acceptable » utilisée dans la définition de la formulation cosmétique telle que décrite ci-dessus, signifie selon la directive du Conseil de la Communauté Economique Européenne N°76/768/CEE du 27 juillet 1976 modifiée par la directive N°93/35/CEE du 14 juin 1993, que ladite formulation comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

L'invention a enfin pour objet un procédé de traitement non thérapeutique de la peau humaine destiné à prévenir l'apparition et/ou à diminuer les cernes et/ou les poches péri-oculaires et/ou le phénomène de jambes lourdes comprenant au moins une étape d'application sur ladite peau humaine d'une quantité efficace de la formulation cosmétique à usage topique telle que définie à précédemment.

Par quantité efficace de composé de formule (I) telle que définie précédemment ou d'une composition (C1) telle que définie précédemment présente dans la formulation cosmétique à usage topique et/ou dans le médicament tels que définis précédemment destinés à prévenir et/ou à traiter l'hypoxie des cellules endothéliales du corps humain, et plus particulièrement destinés à prévenir l'apparition et/ou diminuer les cernes et/ou les poches péri-oculaires et/ou le phénomène de jambes lourdes, on entend pour 100% de la masse de ladite formulation cosmétique à usage topique et/ou du médicament, la quantité comprise entre 0,1% et 5% massique, plus particulièrement entre 0,1% et 3% massique, et encore plus particulièrement entre 0,5% et 2.% massique de composé de formule (I) ou de composition (C1).

Dans le procédé de traitement non thérapeutique tel que décrit ci-dessus, la formulation cosmétique à usage topique est étalée sur la surface de la peau à traiter, puis la peau est massée quelques instants.

Les formulation cosmétique à usage topique objet de la présente invention, se présentent généralement sous forme de solutions aqueuses ou hydro-alcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale, ou sous forme de poudre. Elles peuvent aussi être dispersées ou imprégnées sur du textile ou sur des matériaux non tissés qu'il s'agisse de lingettes, de serviettes en papier ou de vêtements.

De façon générale, le composé de formule (I) ou à la composition (C1) est associé à de nombreux types d'adjuvants ou de principes actifs utilisés dans la formulation cosmétique telle que définie ci-dessus et objet de la présente invention, qu'il s'agisse, de corps gras, de solvants organiques, d'épaississants, de gélifiants, d'adoucissants, d'agents tensioactifs moussants et/ou détergents, d'agents surgraissants, de tensioactifs épaississants et/ou gélifiants, d'antioxydants, d'opacifiants, de stabilisants, de moussants, de parfums, de tensioactifs émulsionnants, d'agents hydrotropes, d'agents plastifiants, d'agents surgraissants, d'agents de texture, de pigments, de séquestrants, de chélateurs, de conservateurs, d'huiles essentielles, de matières colorantes, d'actifs hydrophiles ou lipophiles, d'humectants, de parfums, de filtres solaires minéraux ou organiques, de charges minérales, ou tout autre ingrédient habituellement utilisé en cosmétique.

Comme exemples d'huiles que l'on peut associer au composé de formule (I) ou à la composition (C1) dans les formulations cosmétiques à usage topique objet de la présente invention, on peut citer les huiles minérales telles que l'huile de paraffine, l'huile de vaseline, les isoparaffines ou les huiles blanches minérales, les huiles d'origine animale, telles que le squalène ou le squalane, les huiles végétales, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiées par des aminés, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse que l'on peut associer au composé de formule (I) ou à la composition (C1) dans les formulations cosmétiques à usage topique objet de la présente invention, on peut citer les alcools gras ou les acides gras.

Comme exemple de cires que l'on peut associer au composé de formule (I) ou à la composition (C1) dans les formulations cosmétiques à usage topique objet de la présente invention, on peut citer par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

Comme exemple de polymères épaississants et/ou émulsionnant que l'on peut associer au composé de formule (I) ou à la composition (C1) dans les formulations cosmétiques à usage topique objet de la présente invention, on peut citer par exemple les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique, de chlorure de triméthylaminoéthylacrylate, les hydrocolloïdes d'origine végétale ou biosynthétique, par exemple la gomme de xanthane, la gomme de karaya, les carraghénates, les alginates ; les silicates ; la cellulose et ses dérivés ; l'amidon et ses dérivés hydrophiles ; les polyuréthanes.

Parmi les polymères de type polyélectrolytes que l'on peut associer au composé de formule (I) ou à la composition (C1) dans les formulations cosmétiques à usage topique objet de la présente invention, on peut citer par exemple les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propanesulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL™ EG, SEPIGEL™ 305, SIMULGEL™ NS, SIMULGEL™ 800 et SIMULGEL™ A par la demanderesse.

Comme exemples d'émulsionnants que l'on peut associer au composé de formule (I) ou à la composition (C1) dans les formulations cosmétiques à usage topique objet de la présente invention, on peut citer par exemple les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435 et 2 804 432.

Comme exemples de tensioactifs moussants et/ou détergents que l'on peut associer au composé de formule (I) ou à la composition (C1) dans les formulations cosmétiques à usage topique objet de la présente invention, on peut citer : les tensioactifs anioniques, cationiques, amphotères ou non ioniques topiquement acceptables habituellement utilisés dans ce domaine d'activité.

Parmi les tensioactifs anioniques que l'on peut associer au composé de formule (I) ou à la composition (C1) dans les formulations cosmétiques à usage topique objet de la présente invention, on citera particulièrement les sels de métaux alcalins, les sels de métaux alcalino-terreux, les sels d'ammonium, les sels d'amines, les sels d'aminoalcools des composés suivants : les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkylétherphosphates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcosinates, les acyliséthionates, les N-acyltaurates, les acyllactylates.

Parmi les tensioactifs anioniques que l'on peut associer au composé de formule (I) ou à la composition (C1) dans les formulations cosmétiques à usage topique objet de la présente invention, on citera également les dérivés N-acylés d'acides aminés, de peptides, de protéines dont la chaîne acyle comporte de 8 à 16 atomes de carbone ; les sels d'acides gras, les sels d'acides d'huile de coprah éventuellement hydrogénée.

Parmi les tensioactifs amphotères que l'on peut associer au composé de formule (I) ou à la composition (C1) dans les formulations cosmétiques à usage topique objet de la présente invention, on citera particulièrement les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques que l'on peut associer au composé de formule (I) ou à la composition (C1) dans les formulations cosmétiques à usage topique objet de la présente invention, on citera particulièrement les dérivés d'ammoniums quaternaires.

Parmi les tensioactifs non ioniques que l'on peut associer au composé de formule (I) ou à la composition (C1) dans les formulations cosmétiques à usage topique objet de la présente invention, on citera particulièrement les alkylpolyglycosides dont la chaîne alkyle comporte de 8 à 16 atomes de carbone, les dérivés d'huile de ricin, les polysorbates, les amides de coprah, les N-alkylamines, les oxydes d'amines.

Comme exemples d'agents de texture que l'on peut associer au composé de formule (I) ou à la composition (C1) dans les formulations cosmétiques à usage topique objet de la présente invention, on peut citer par exemple des dérivés N-acylés d'acides aminés, comme par exemple la lauroyl lysine commercialisée sous l'appellation AMINOHOPE™LL par la société AJINOMOTO, l'octenyl starch succinate commercialisé sous l'appellation DRYFLO™ par la société NATIONAL STARCH, le myristyl polyglucoside commercialisé par SEPPIC sous l'appellation MONTANOV 14, les fibres de cellulose, les fibres de coton, les fibres de chitosane, le talc, la séricite, le mica.

Comme exemples d'agents opacifiants et/ou nacrants que l'on peut associer au composé de formule (I) ou à la composition (C1) dans les formulations cosmétiques à usage topique objet de la présente invention, on peut citer par exemple le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras.

Comme exemples de tensioactifs épaississants et/ou gélifiants que l'on peut associer au composé de formule (I) ou à la composition (C1) dans les formulations cosmétiques à usage topique objet de la présente invention, on peut citer :
- Les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120.
- Les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141.
- Les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples de filtres solaires que l'on peut associer au composé de formule (I) ou à la composition (C1) dans les formulations cosmétiques à usage topique objet de la présente invention, on peut citer tous ceux figurant dans la directive cosmétique 76/768/CEE modifiée annexe VII.

Comme exemples de principe actif que l'on peut associer au composé de formule (I) ou à la composition (C1) dans les formulations cosmétiques à usage topique objet de la présente invention, on peut citer les composés ayant une action éclaircissante ou dépigmentante tels que par exemple l'arbutine, l'acide kojique, l'hydroquinone, l'acide ellagique, la vitamine C, le magnésium ascorbyl phosphate, les extraits de polyphénols, les dérivés de polyphénols glycosylés comme le Rosmarinyl glucoside, les extraits de raisin, les extraits de pin, les extraits de vin, les extraits d'olives, les extraits de mare, les protéines N-acylées, les peptides N-acylés, les acides aminés N-acylés, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol), l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, la vitamine E et ses dérivés, les minéraux, les enzymes, les coenzymes, comme, le Coenzyme Q10, les hormones ou "hormone like", les extraits de soja par exemple, la Raffermine™, les extraits de blé par exemple la Tensine™ ou la Gliadine™, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action amincissante comme la caféine ou ses dérivés, comme l'extrait de quinoa commercialisé sous l'appellation ADIPOLESS™, comme l'extrait de pruche canadienne commercialisé sous l'appellation SERENIKS™ 207, comme la composition comprenant du Lauroyl Proline commercialisée sous l'appellation ADIPOSLIM™, les actifs ayant une activité anti-microbienne ou une action purifiante vis à vis des peaux grasses tels que le LIPACIDE™ PVB, les actifs ayant une propriété énergisante ou stimulante comme le SEPITONIC™ M3 ou le Physiogényl™ le panthénol et ses dérivés comme le SEPICAP™ MP, les actifs anti-âge comme le SEPILIFT™ DPHP, le LIPACIDE™ PVB, le SEPIVINOL™, le SEPIVITAL™, les actifs hydratants comme le SEPICALM™ S, le SEPICALM™ VG et le SEPILIFT™ DPHP, les actifs anti-âge " anti-photo vieillissement ", les actifs protecteurs de l'intégrité de la jonction dermo-épidermique, les actifs augmentant la synthèse des composants de la matrice extracellulaire, les actifs ayant une activité amincissante comme la caféine, la théophylline, l'AMPc, le thé vert, la sauge, le ginko biloba, le lierre, le marron d'inde, le bambou, le ruscus, le petit houx, la centella asiatica, la bruyère, l'ulmaine, le fucus, le romarin, la saule, des actifs créant une sensation de « chauffe » sur la peau comme les activateurs de la microcirculation cutanée (exemple des nicotinates) ou des produits créant une sensation de « fraîcheur » sur la peau (exemple du menthol et des dérivés).

L'invention a aussi pour objet un composé de formule (I) ou une composition (C1) telles que définies précédemment, pour la mise en oeuvre d'une méthode de traitement thérapeutique du corps humain ou animal etplus particulièrement un composé de formule (I) ou une composition (C1) telles que définies précédemment, pour son utilisation dans une méthode de traitement thérapeutique de l'hypoxie des cellules endothéliales du corps humain ou animal, et plus particulièrement une méthode de traitement thérapeutique des cernes, des poches péri-oculaires et/ou des jambes lourdes.

L'étude expérimentale suivante illustre l'invention sans toutefois la limiter.

### Exemples de préparation de composés de formule (I) selon l'invention

### Exemple 1 : préparation d'une composition A comprenant les composés de formules (Ia1) et (Ib1).

On introduit 400,0 grammes de proline soit un équivalent molaire dans un mélange de 1600 grammes constitué par 1440 grammes d'eau et de 160 grammes d'isopropanol compris dans un réacteur en verre à double enveloppe, dans laquelle circule un fluide caloporteur, muni d'une agitation efficace et d'un dispositif de barbotage d'azote par fond de réacteur, à une température de 20°C. Le pH du milieu ainsi préparé est ajusté à une valeur de pH de 10 par ajout d'une solution de soude à 30%. 450 grammes de chlorure d'octanoyle soit 0,8 équivalent molaire sont ensuite ajoutés progressivement sur le milieu à une température comprise entre 20°C et 30°C, de façon à maitriser l'exothermie ; une solution de soude à 30% est parallèlement ajoutée au milieu de façon à maintenir la valeur du pH du milieu à une valeur comprise entre 10 et 10,5.

A la fin de l'addition du chlorure d'octanoyle, le milieu réactionnel est maintenu sous agitation pendant une durée de 2 heures. Le milieu réactionnel est ensuite porté à une température de 70°C sous agitation et une quantité de 724 grammes d'une solution acide d'acide phosphorique à 75% est ensuite progressivement introduite de façon à obtenir une valeur de pH du milieu réactionnel d'environ 2,0. L'agitation est arrêtée et la phase aqueuse du milieu décantée est ensuite soutirée. La phase organique restant dans le réacteur est ensuite lavée par une quantité de 3000 grammes d'eau à température ambiante sous agitation. La phase aqueuse est soutirée par le fond du réacteur et la phase de lavage telle que décrite précédemment est répétée une fois supplémentaire. A l'issu du lavage, la phase organique est séchée par distillation sous vide de l'eau résiduelle.

Une quantité de 90 grammes d'isosorbide soit un équivalent molaire d'isosorbide est introduite dans le réacteur comprenant 161 grammes du milieu réactionnel séché, agité et porté à une température de 120°C. Lorsque le mélange est bien dispersé, une quantité de 0,6 grammes d'acide sulfurique à 98% est introduite dans le réacteur, et le mélange résultant est porté à une température de 125°C, sous un vide partiel avec un barbotage d'azote régulier introduit par le fond du réacteur. Le mélange réactionnel est ensuite maintenu pendant une durée de 24 heures sous agitation et à 125°C, puis neutralisé par ajout d'une solution de soude à 30% de façon à obtenir une valeur de pH de 5% du milieu réactionnel comprise entre 3,0 et 6,0. Le milieu réactionnel est ensuite vidangé et les caractéristiques analytiques de la composition A mesurées sont les suivantes :
Indice acide (selon NFT 60-204) = 85,8
pH 5% de la composition A dans l'eau (selon la méthode NFT 73-206) = 3,3
Indice d'Hydroxyle (selon U.S. Pharmacopeia XXI NF XVI 01/011985) = 214,7
Indice d'ester (calculé par la différence entre l'indice de saponification mesuré selon la méthode NFT 60-110 et l'indice d'acide mesuré selon la méthode NFT 60-204) = 65,3

### Exemple 2: préparation d'une composition B comprenant les composés de formules (Ia2) et (Ib2).

Le mode opératoire du procédé décrit dans l'exemple 1 est mis en oeuvre pour un équivalent molaire de proline, 0,8 équivalent molaire de chlorure d'undécylènoyle et un équivalent molaire d'isosorbide pour obtenir la composition B dont les caractéristiques analytiques sont les suivantes :
Indice acide (selon NFT 60-204) = 78,4
pH 5% de la composition B dans l'eau (selon la méthode NFT 73-206) = 4,2
Indice d'Hydroxyle (selon U.S. Pharmacopeia XXI NF XVI 01/011985) = 191,7
Indice d'ester (calculé par la différence entre l'indice de saponification mesuré selon la méthode NFT 60-110 et l'indice d'acide mesuré selon la méthode NFT 60-204) = 67,8

### Exemple 3: préparation d'une composition D comprenant les composés de formules (Ia3) et (Ib3).

Le mode opératoire du procédé décrit dans l'exemple 1 est mis en oeuvre pour un équivalent molaire de proline, 0,8 équivalent molaire de chlorure d'hexadécanoyle et un équivalent molaire d'isosorbide pour obtenir la composition D dont les caractéristiques analytiques sont les suivantes :
Indice acide (selon NFT 60-204) = 64,1
pH 5% de la composition D dans l'eau (selon la méthode NFT 73-206) = 4,8
Indice d'Hydroxyle (selon U.S. Pharmacopeia XXI NF XVI 01/011985) = 166,9
Indice d'ester (calculé par la différence entre l'indice de saponification mesuré selon la méthode NFT 60-110 et l'indice d'acide mesuré selon la méthode NFT 60-204) = 55,4.

### Exemple 4: préparation d'une composition E comprenant les composés de formules (Ia4) et (Ib4).

Le mode opératoire du procédé décrit dans l'exemple 1 est mis en oeuvre pour un équivalent molaire de valine, 0,8 équivalent molaire de chlorure d'hexadécanoyle et un équivalent molaire d'isosorbide pour obtenir la composition D dont les caractéristiques analytiques sont les suivantes :
Indice acide (selon NFT 60-204) = 12,3
pH 5% de la composition E dans l'eau (selon la méthode NFT 73-206) = 7,5
Indice d'Hydroxyle (selon U.S. Pharmacopeia XXI NF XVI 01/011985) = 100,2
Indice d'ester (calculé par la différence entre l'indice de saponification mesuré selon la méthode NFT 60-110 et l'indice d'acide mesuré selon la méthode NFT 60-204) = 96,2

### Exemple 5: préparation d'une composition F comprenant les composés de formules (Ia5) et (Ib5).

Le mode opératoire du procédé décrit dans l'exemple 1 est mis en oeuvre pour un équivalent molaire de valine, 0,8 équivalent molaire de chlorure d'octanoyle et un équivalent molaire d'isosorbide pour obtenir la composition F dont les caractéristiques analytiques sont les suivantes :
Indice acide (selon NFT 60-204) = 43,7
pH 5% de la composition F dans l'eau (selon la méthode NFT 73-206) = 4,0
Indice d'Hydroxyle (selon U.S. Pharmacopeia XXI NF XVI 01/011985) = 181,4
Indice d'ester (calculé par la différence entre l'indice de saponification mesuré selon la méthode NFT 60-110 et l'indice d'acide mesuré selon la méthode NFT 60-204) = 99,3

### Evaluation de l'effet de composés et de compositions selon l'invention sur la production d'ATP intracellulaire dans des cultures de cellules endothéliales ayant subi un stress oxydant.

### Protocole

Des cellules HUVEC (Human Umbilical Vein Endothelial Cells en langue anglaise) au passage R3 sont ensemencées à 2000 cellules/puits dans des plaques comprenant 96 puits.

Les cellules sont ensuite cultivées en milieu EGM-2 (Endothelial Growth Médium en langue anglaise), commercialisé par la société Lonza, pendant 7 jours à une température de 37°C sous 5% de CO₂.

Les milieux de culture sont alors remplacés par du milieu EGM-2, contenant les dilutions des composés et des compositions à tester.

Des témoins (T) sont également préparés en remplaçant des milieux de culture par le seul milieu EGM-2.

Les cellules présentes dans le milieu EGM-2 et associées aux dilutions des composés et des compositions à tester, ainsi que les témoins (T) sont ensuite incubés pendant une durée de 24 heures à une température de 37°C.

Suite à cette incubation, les milieux des cellules associées aux dilutions des composés et des compositions à tester et les milieux des témoins (T) sont remplacés par un milieu d'EGM-2 supplémenté en eau oxygénée à une concentration de 0,8 millimoles par litre.

On notera par la suite «témoins (T1) » les milieux des témoins (T) supplémentés en eau oxygénée et « témoins (T) » les milieux des témoins (T) non supplémentés en eau oxygénée.

Les cellules associées aux dilutions des composés et des compositions à tester, les témoins (T) et les témoins (T1) sont ensuite incubés pendant 10 minutes à une température de 37°C, puis rincés avec du PBS (Phosphate Buffer Saline).

La quantité d'ATP intracellulaire et la quantité de protéines produites par les cellules associées aux dilutions des composés et des compositions à tester, les témoins (T) et les témoins (T1) sont évaluées après lyse des tapis moléculaires en présence d'un tampon de lyse.

La quantité d'ATP est quantifiée par la méthode luminométrique, mise en oeuvre par l'intermédiaire d'un lecteur de plaques luminomètre de marque FLUOROSKAN ASCENT FL™ commercialisé par la société LABSYSTEMS, et la quantification des protéines est réalisée à l'aide de la méthode BCA. Cette dernière méthode permet de normaliser les quantités d'ATP dosées et d'évaluer la cytotoxycité de chaque condition expérimentale.

Un seuil de cytotoxicité a été fixé à 80% du groupe témoin.

Les résultats sont exprimés en millimole d'ATP produite par milligramme de protéines produites et l'étude statistique des résultats a été réalisée à l'aide du test T de Student bilatéral à variance inégale.

Les effets évalués sur les cellules associées aux dilutions des composés et des compositions à tester, les témoins (T) et les témoins (T1) ont été réalisés sur deux expériences indépendantes et les résultats présentés correspondent à la moyenne des deux essais.

Pour chaque condition expérimentale, les statistiques les plus restrictives ont été appliquées. Les résultats obtenus sont consignés dans le Tableau 1 suivant :

**Tableau 1**

| Composition testée | Concentration (% p/v) par rapport à l'extrait sec) | Quantité d'ATP produite (mmoles d'ATP/mg de protéines) |
|---|---|---|
| Témoin (T) | - | 0,045 |
| Témoin (T1) | - | 0,016 |
| Composition A | 0,0001% | 0,021 |
| Composition B | 0,000025% | 0,022 |
| Composition B | 0,00005% | 0,023 |
| Composition D | 0,00005% | 0,025 |
| Composition D | 0,0005% | 0,022 |
| Composition E | 0,00005% | 0,020 |
| Composition F | 0,00005% | 0,021 |
| Composition F | 0,0005% | 0,023 |

Le stress oxydant procuré par l'ajout d'eau oxygénée sur les cellules témoins (T) induit une diminution de 64% de l'ATP produite par les cellules endothéliales (cellules témoins (T1). Ce résultat valide ainsi les conditions du test expérimental mis en oeuvre.

Lorsque les cellules endothéliales sont associées à la composition A, la quantité d'ATP produite par lesdites cellules endothéliales est en augmentation de 31,25% par rapport aux cellules témoins (T1) soumises au même stress oxydant.

Lorsque les cellules endothéliales sont associées aux compositions A à F, les quantités d'ATP produites par lesdites cellules endothéliales est en augmentation de 25% (pour la composition E) à 56,2% (pour la composition D testée à 0, 000 05%) par rapport aux cellules témoins (T1) soumises au même stress oxydant.

Aucune des compositions testées n'a induit de cytotoxicité significative selon le test BCA mis en oeuvre avec le kit « BC-assay » commercialisé par la société Interchim.

Il en résulte que les compositions selon l'invention comprenant les composés selon l'invention permettent de ralentir la baisse de la production d'ATP par les cellules endothéliales soumises à des stress oxydants.

### III- Références bibliographiques citées dans la description

(1) Chang et al.: "Aging and survival of cutaneous microvasculature", J. Invest Dermatol. 2002 May;118(5):752-8.
(2) Chung et al.: "Differential effects of photoaging vs intrinsic aging on the vascularization of human skin" Arch. Dermatol. 2002 Nov;138(11):1437-42.
(3) Toyoda et al.: "Ultrastructural characterization of microvasculature in photoaging" J. Dermatol Sci. 2001 Aug;27 Suppl 1:S32-41.
(4) Janssens, « effect of venotropic drugs on the respiratory activity of isolated mitochondria and in endothelial cells », in British Journal of Pharmacology (2000) 130, 1513-1524
(5) M. Okada et al., J.Appl.Polym.Sci., 2001, 81(11), pages 2721-2734.
(6) Z. Gomurashvili et al., J.Macromol.Sci., Pure Appl.Chem., 2000, A37(3), 215-227

## Revendications

1. Composé de formule (I) : 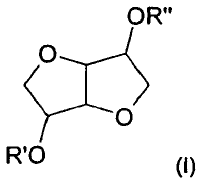 dans laquelle R' et R", identiques ou différents, représentent :
- Soit un atome d'hydrogène ;
- Soit un radical monovalent de formule (IIa) : 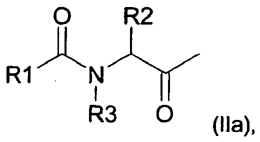
dans laquelle :
- R1 représente un radical aliphatique, saturé ou insaturé, linéaire ou ramifié, comportant de 7 à 30 atomes de carbone,
- R2 représente un atome d'hydrogène ou un radical choisi parmi les radicaux méthyle, isopropyle, isobutyle, 1-méthyl propyle, hydroxyméthyle, 1-hydroxy éthyle, thiométhyle, 2-méthylthio éthyle, 4-aminobutyle, 3-guanidino propyle, 3-uréido propyle, (1-amino carbonyl) méthyle, carboxy méthyle, 2-carboxy éthyle, 2-(amino carbonyl) éthyle, benzyle, 4-hydroxy benzyle, 3,4-dihydroxy benzyle, [1H-indol-3-yl] méthyle, (1H-imidazol-4-yl) méthyle, 3-amino propyle, et
- R3 représente un atome d'hydrogène ou un radical méthyle ;
- Soit un radical monovalent de formule (IIb) : 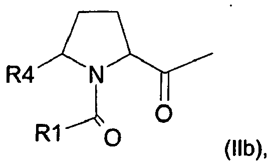
dans laquelle :
- R1 est tel que défini dans la formule (IIa) et
- R4 représente un atome d'hydrogène ou un radical hydroxy, étant entendu qu'au moins un des radicaux R' ou R" ne représente pas un atome d'hydrogène et que lorsqu'aucun des radicaux R' et R" ne représente un atome d'hydrogène, R' et R" sont identiques.

2. Composé de formule (I) telle que définie à la revendication 1, dans laquelle le radical R₁ comporte de 7 à 22 atomes de carbone.

3. Composé de formule (I) telle que définie à l'une ou quelconque des revendications 1 ou 2, dans laquelle les radicaux R' et R", identiques ou différents, représentent :
- Soit un atome d'hydrogène ;
- Soit un radical monovalent de formule (IIa), dans laquelle R1 et R3 sont tels que définis précédemment et R2 représente un radical choisi parmi les radicaux méthyle, isopropyle, isobutyle, 1-méthyl propyle ou benzyle,
- Soit un radical monovalent de formule (IIb), dans laquelle R1 est tel que défini précédemment et R4 représente un atome d'hydrogène.

4. Composé de formule (la) : 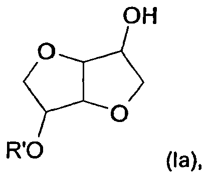 correspondant à la formule (I) telle que définie à l'une ou quelconque des revendications 1 à 3, dans laquelle R" représente un atome d'hydrogène.

5. Composé de formule (Ib) : 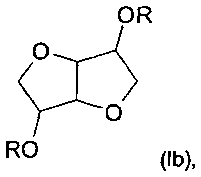 correspondant à la formule (I) telle que définie à l'une ou quelconque des revendications 1 à 3, dans laquelle les radicaux R' et R" sont identiques et sont représentés par le radical R.

6. Procédé de préparation d'un composé de formule (I) telle que définie à l'une ou quelconque des revendications 1 à 5, comprenant :
- Une étape a) d'estérification :
- Soit d'un composé de formule (IIIa) : 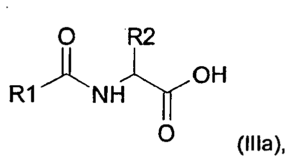 dans laquelle R1 et R2 sont tels que définis dans la formule (IIa),
- Soit d'un composé de formule (IIIb) : 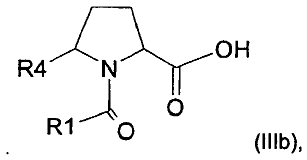 dans laquelle R₁ et R4 sont tels que définis pour la formule (IIb), avec l'isosorbide de formule (IV) : 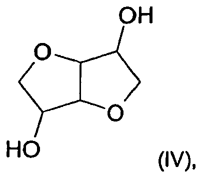 pour obtenir, soit le composé de formule (la), soit le composé de formule (Ib), soit un mélange (M) du composé de formule (la) et du composé de formule (Ib) ; et si nécessaire ou si désiré,
- Une étape b) de séparation des composés de formule (la) et de formule (Ib), à partir du dudit mélange (M) obtenu à l'étape (a).

7. Procédé de préparation d'un composé de formule (I) telle que définie à l'une ou quelconque des revendications 1 à 5 comprenant :
- Une étape a1) d'estérification, soit d'un composé de formule (IIIa) 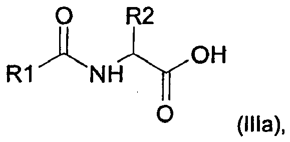 dans laquelle R1 et R2 sont tels que définis dans la formule (IIa), soit d'un composé de formule (IIIb) 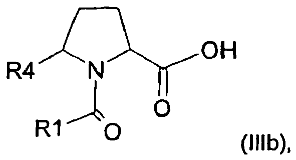 dans laquelle R₁ et R4 sont tels que définis pour la formule (IIb), avec un alcool de formule (V) :
R5-OH (V),
dans laquelle R5 représente un radical aliphatique linéaire comportant de 1 à 4 atomes de carbone, pour former :
- Soit un composé de formule (VIa) : 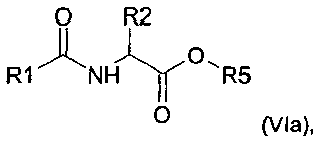 dans laquelle R1, R2 et R5 sont tels que définis précédemment,
- Soit un composé de formule (VIb) : 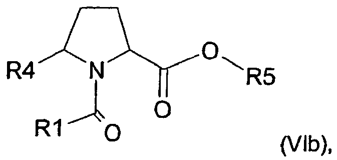 dans laquelle R1, R4 et R5 sont tels que définis précédemment ;
- Une étape a2) de trans-estérification du composé de formule (Via) ou du composé de formule (Vlb) obtenu à l'étape a1), par réaction avec l'isosorbide de formule (IV), pour obtenir, soit le composé de formule (Ia), soit le composé de formule (Ib), soit un mélange (M) du composé de formule (la) et du composé de formule (Ib) ; et si nécessaire ou si désiré,
- La mise en oeuvre de l'étape b) telle que définie à la revendication 6.

8. Composition (C1) comprenant pour 100% de sa masse :
- De 99% massique à 20% massique d'au moins un composé de formule (la) 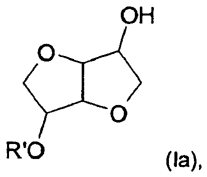 correspondant à la formule (I) telle que définie à l'une ou quelconque des revendications 1 à 3, dans laquelle R" représente un atome d'hydrogène ;
- De 1% massique à 80% massique d'au moins un composé de formule (Ib) : 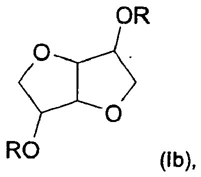 correspondant à la formule (I) telle que définie à l'une ou quelconque des revendications 1 à 3, dans laquelle les radicaux R' et R" sont identiques et sont représentés par le radical R.

9. Formulation cosmétique à usage topique **caractérisée en ce qu'**elle comprend au moins un excipient cosmétiquement acceptable et une quantité efficace du composé de formule (I) telle que définie à l'une ou quelconque des revendications 1 à 5, ou de la composition (C1) telle que définie à la revendication 8.

10. Composé de formule (I) telle que définie à l'une quelconque des revendications 1 à 5, ou composition (C1) telle que définie à la revendication 8, pour la mise en oeuvre d'une méthode de traitement thérapeutique du corps humain ou animal.

11. Composé de formule (I) telle que définie à l'une quelconque des revendications 1 à 5, ou composition (C1) telle que définie à la revendication 8, pour son utilisation dans une méthode de traitement thérapeutique de l'hypoxie des cellules endothéliales du corps humain ou animal.

12. Composé de formule (I) telle que définie à l'une quelconque des revendications 1 à 5, ou composition (C1) telle que définie à la revendication 8, pour son utilisation dans une méthode de traitement thérapeutique telle que définie à la revendication 11 destinée au traitement thérapeutique des cernes, des poches péri-oculaires et/ou des jambes lourdes.

## Patentansprüche

1. Verbindung der Formel (I): 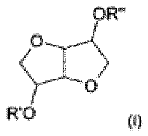 worin R' und R", die identisch oder verschieden sind, Folgendes darstellen:
- entweder ein Wasserstoffatom;
- oder ein einwertiges Radikal der Formel (IIa): 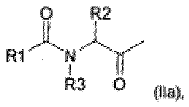 worin:
- R1 ein geradkettiges oder verzweigtes, gesättigtes oder ungesättigtes aliphatisches Radikal mit 7 bis 30 Kohlenstoffatomen darstellt,
- R2 ein Wasserstoffatom oder ein Radikal darstellt, das ausgewählt ist aus den Methyl-, Isopropyl-, Isobutyl-, 1-Methylpropyl-, Hydroxymethyl-, 1-Hydroxyethyl-, Thiomethyl-, 2-Methylthioethyl-, 4-Aminobutyl-, 3-Guanidinpropyl-, 3-Ureidopropyl-, (1-Aminocarbonyl)-Methyl-, Carboxymethyl-, 2-Carboxyethyl-, 2-(Aminocarbonyl)-Ethyl-, Benzyl-, 4-Hydroxybenzyl-, 3,4-Dihydroxybenzyl-, [1H-Indol-3-yl]-Methyl-, (1H-Imidazol-4-yl)-Methyl-, 3-Aminopropyl-Radikalen, und
- R3 ein Wasserstoffatom oder ein Methyl-Radikal darstellt;
- oder ein einwertiges Radikal der Formel (IIb): 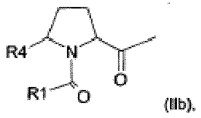
worin:
- R1 wie in der Formel (IIa) definiert ist und
- R4 ein Wasserstoffatom oder ein Hydroxy-Radikal darstellt;
wobei mindestens einer der Radikale R' oder R" kein Wasserstoffatom darstellt und, wenn keines der Radikale ein Wasserstoffatom darstellt, R' und R" identisch sind.

2. Verbindung der Formel (I) nach Anspruch 1, worin das Radikal R₁ 7 bis 22 Kohlenstoffatome aufweist.

3. Verbindung der Formel (I) nach einem der Ansprüche 1 oder 2, worin R' und R", die identisch oder verschieden sind, Folgendes darstellen:
- entweder ein Wasserstoffatom;
- oder ein einwertiges Radikal der Formel (IIa), worin R1 und R3 wie vorstehend definiert sind und R2 ein Radikal darstellt, das ausgewählt ist aus den Methyl-, Isopropyl-, Isobutyl-, 1-Methylpropyl- oder Benzyl-Radikalen,
- oder ein einwertiges Radikal der Formel (IIb), worin R1 wie vorstehend definiert ist und R4 ein Wasserstoffatom darstellt.

4. Verbindung der Formel (la): 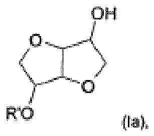 entsprechend der Formel (I) nach einem der Ansprüche 1 bis 3, worin R" ein Wasserstoffatom darstellt.

5. Verbindung der Formel (Ib): 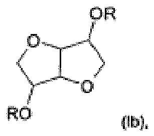 entsprechend der Formel (I) nach einem der Ansprüche 1 bis 3, worin die Radikale R' und R" identisch sind und durch das Radikal R dargestellt werden.

6. Verfahren zum Herstellen einer Verbindung der Formel (I) nach einem der Ansprüche 1 bis 5, umfassend:
- einen Schritt a) der Veresterung:
- entweder einer Verbindung der Formel (IIIa): 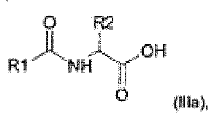 worin R1 und R2 wie in der Formel (IIa) definiert sind,
- oder einer Verbindung der Formel IIIb): 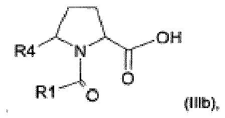 worin R₁ und R4 wie in Formel (IIb) definiert sind, mit einem Isosorbid der Formel (IV): 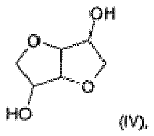 um entweder die Verbindung der Formel (la) oder die Verbindung der Formel (Ib) oder ein Gemisch (M) der Verbindung der Formel (la) und der Verbindung der Formel (Ib) zu erhalten; und, falls notwendig oder erwünscht,
- einen Schritt b) der Trennung der Verbindungen der Formel (la) und der Formel (Ib) aus dem Gemisch (M), das in Schritt (a) erhalten wurde.

7. Verfahren zum Herstellen einer Verbindung der Formel (I) nach einem der Ansprüche 1 bis 5, umfassend:
- einen Schritt a1) der Veresterung entweder einer Verbindung der Formel (IIIa): 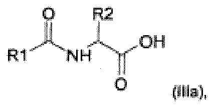 worin R1 und R2 wie in Formel (IIa) definiert sind, oder einer Verbindung der Formel (IIIb): 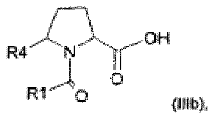 worin R1 und R4 wie in Formel (IIb) definiert sind, mit einem Alkohol der Formel (V):
R5-OH (V)
worin R5 ein lineares aliphatisches Radikal mit 1 bis 4 Kohlenstoffatomen darstellt, zur Bildung:
- entweder einer Verbindung der Formel (VIa): 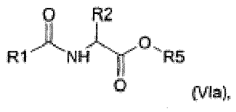 worin R1, R2 und R5 wie vorstehend definiert sind,
- oder einer Verbindung der Formel (Vlb): 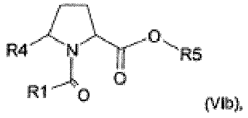 worin R1, R4 und R5 wie vorstehend definiert sind;
- einen Schritt a2) der Umesterung der Verbindung der Formel (VIa) oder der Verbindung der Formel (Vlb), die in Schritt a1) erhalten wurde, zur Reaktion mit dem Isosorbid der Formel (IV), um entweder die Verbindung der Formel (la) oder die Verbindung der Formel (Ib) oder ein Gemisch (M) der Verbindung der Formel (Ia) und der Verbindung der Formel (Ib) zu erhalten; und, falls notwendig oder erwünscht,
- die Durchführung von Schritt b) nach Anspruch 6.

8. Zusammensetzung (C1), die pro 100 % ihrer Masse Folgendes enthält:
- 99 Massen-% bis 20 Massen-% wenigstens einer Verbindung der Formel (la): 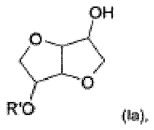 entsprechend der Formel (I) nach einem der Ansprüche 1 bis 3, worin R" ein Wasserstoffatom darstellt;
- 1 Massen-% bis 80 Massen-% wenigstens einer Verbindung der Formel (Ib): 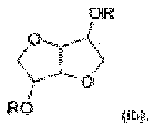 entsprechend der Formel (I) nach einem der Ansprüche 1 bis 3, worin die Radikale R' und R" identisch sind und durch das Radikal R dargestellt werden.

9. Kosmetische Formulierung zur topischen Verwendung, **dadurch gekennzeichnet, dass** sie wenigstens einen kosmetisch verträglichen Träger und eine wirksame Menge der Verbindung der Formel (I) nach einem der Ansprüche 1 bis 5 oder der Zusammensetzung (C1) nach Anspruch 8 enthält.

10. Verbindung der Formel (I) nach einem der Ansprüche 1 bis 5 oder Zusammensetzung (C1) nach Anspruch 8 zur Durchführung eines Verfahrens zur therapeutischen Behandlung des menschlichen oder tierischen Körpers.

11. Verbindung der Formel (I) nach einem der Ansprüche 1 bis 5 oder Zusammensetzung (C1) nach Anspruch 8, für ihre Verwendung in einem Verfahren zur therapeutischen Behandlung der Hypoxie der endothelialen Zellen des menschlichen oder tierischen Körpers.

12. Verbindung der Formel (I) nach einem der Ansprüche 1 bis 5 oder Zusammensetzung (C1) nach Anspruch 8 für ihre Verwendung in einem Verfahren zur therapeutischen Behandlung nach Anspruch 11, das zur therapeutischen Behandlung von Augenringen, Tränensäcken und/oder schweren Beinen bestimmt ist.

## Claims

1. Compound of formula (I): 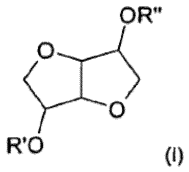 wherein R' and R", which are identical or different, represent:
- either a hydrogen atom;
- or a monovalent radical of formula (IIa): 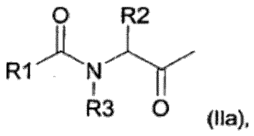
wherein:
- R1 represents a saturated or unsaturated, linear or branched aliphatic radical, comprising 7 to 30 carbon atoms,
- R2 represents a hydrogen atom or a radical selected from the methyl, isopropyl, isobutyl, 1-methyl propyl, hydroxymethyl, 1-hydroxy ethyl, thiomethyl, 2-methylthio ethyl, 4-aminobutyl, 3-guanidino propyl, 3-ureido propyl, (1-amino carbonyl)methyl, carboxy methyl, 2-carboxy ethyl, 2-(amino carbonyl)ethyl, benzyl, 4-hydroxy benzyl, 3,4-dihydroxy benzyl, [1 H-indol-3-yl]methyl, (1 H-imidazol-4-yl)methyl and 3-amino propyl radicals, and
- R3 represents a hydrogen atom or a methyl radical;
- or a monovalent radical of formula (IIb): 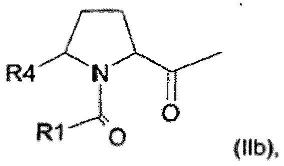
wherein:
- R1 is as defined in formula (IIa) and
- R4 represents a hydrogen atom or a hydroxy radical,
it being understood that at least one of the radicals R' or R" does not represent a hydrogen atom and that when the radicals R' and R" do not represent a hydrogen atom, R' and R" are identical.

2. Compound of formula (I) according to claim 1, wherein the radical R₁ comprises 7 to 22 carbon atoms.

3. Compound of formula (I) according to either claim 1 or claim 2, wherein the radicals R' and R", which are identical or different, represent:
- either a hydrogen atom,
- or a monovalent radical of formula (IIa), wherein R1 and R3 are as defined above and R2 represents a radical selected from the methyl, isopropyl, isobutyl, 1-methyl propyl and benzyl radicals,
- or a monovalent radical of formula (IIb), wherein R1 is as defined above and R4 represents a hydrogen atom.

4. Compound of formula (Ia): 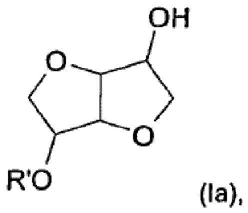 corresponding to formula (I) according to any of claims 1 to 3, wherein R" represents a hydrogen atom.

5. Compound of formula (Ib): 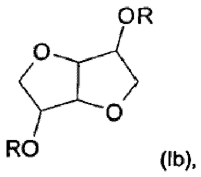 corresponding to formula (I) according to any of claims 1 to 3, wherein the radicals R' and R" are identical and are represented by the radical R.

6. Method for preparing a compound of formula (I) according to any of claims 1 to 5, comprising:
- a step a) of esterification:
- either of a compound of formula (IIIa): 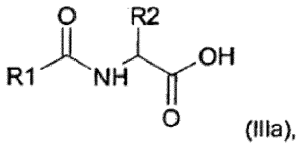 wherein R1 and R2 are as defined in formula (IIa),
- or a compound of formula (IIIb): 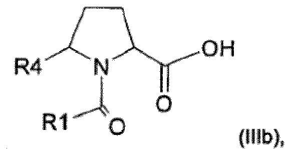 wherein R₁ and R4 are as defined for formula (IIb) with isosorbide of formula (IV): 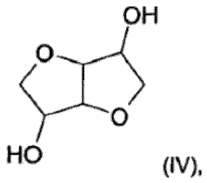 to obtain either the compound of formula (Ia), or the compound of formula (Ib),
or a mixture (M) of the compound of formula (Ia) and the compound of formula (Ib); and if necessary or if desired,
- a step b) of separating the compounds of formula (Ia) and of formula (Ib), starting from said mixture (M) obtained in step (a).

7. Method for preparing a compound of formula (I) according to any of claims 1 to 5, comprising:
- a step a1) of esterification either of a compound of formula (IIIa) 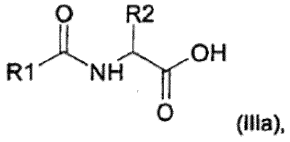 wherein R1 and R2 are as defined in formula (IIa), or of a compound of formula (IIIb) 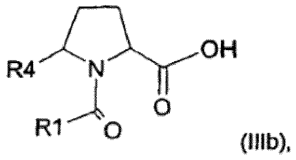 wherein R₁ and R4 are as defined for formula (IIb), with an alcohol of formula (V):
R5-OH (V).
wherein R5 represents a linear aliphatic radical comprising 1 to 4 carbon atoms, to form:
- either a compound of formula (VIa): 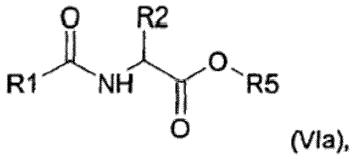 wherein R1, R2 and R5 are as defined above,
- or a compound of formula (Vlb): 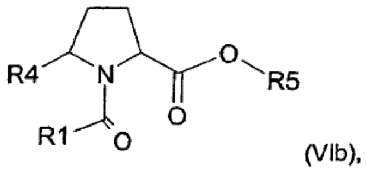 wherein R1, R4 and R5 are as defined above;
- a step a2) of trans-esterification of the compound of formula (VIa) or of the compound of formula (VIb) obtained in step a1), by reaction with the isosorbide of formula (IV), to obtain either the compound of formula (Ia) or the compound of formula (Ib), or a mixture (M) of the compound of formula (Ia) and the compound of formula (Ib); and if necessary or if desired,
- implementation of step b) according to claim 6.

8. Composition (C1) comprising for 100 % of its weight:
- from 99 % by weight to 20 % by weight of at least one compound of formula (Ia) 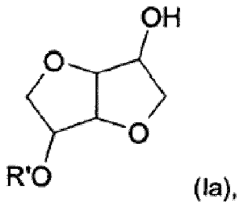 corresponding to formula (I) according to any of claims 1 to 3, wherein R" represents a hydrogen atom,
- from 1 % by weight to 80 % by weight of at least one compound of formula (Ib): 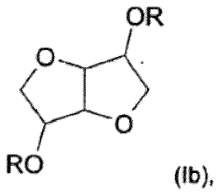 corresponding to formula (I) according to any of claims 1 to 3, wherein the radicals R' and R" are identical and are represented by the radical R.

9. Cosmetic formulation for topical use, **characterised in that** it comprises at least one cosmetically acceptable excipient and an effective amount of the compound of formula (I) according to any of claims 1 to 5, or of the composition (C1) according to claim 8.

10. Compound of formula (I) according to any of claims 1 to 5, or a composition (C1) according to claim 8, for the implementation of a method for therapeutic treatment of the human or animal body.

11. Compound of formula (I) according to any of claims 1 to 5, or a composition (C1) according to claim 8, for use in a method for therapeutic treatment of hypoxia of the endothelial cells of the human or animal body.

12. Compound of formula (I) according to any of claims 1 to 5, or a composition (C1) according to claim 8, for use in a therapeutic treatment method according to claim 11, intended for the therapeutic treatment of dark circles or bags under the eyes and/or heavy legs.
